(19) Europäisches Patentamt
European Patent Office
Office européen des brevets

(11) Veröffentlichungsnummer: **0 098 478**
**B1**

(12) # EUROPÄISCHE PATENTSCHRIFT

(45) Veröffentlichungstag der Patentschrift: **06.11.85**

(21) Anmeldenummer: **83106171.8**

(22) Anmeldetag: **24.06.83**

(51) Int. Cl.⁴: **C 07 D 498/04,** C 07 D 498/14, C 07 D 513/10, C 07 D 405/06, C 07 D 319/06 // (C07D498/04, 205:00, 265:00)

(54) **Verfahren zur Herstellung von 7-Amino-1-dethia-1-oxa-3-hydroymethyl-cephem-4-carbonsäuren.**

(30) Priorität: **06.07.82 DE 3225269**

(43) Veröffentlichungstag der Anmeldung:
**18.01.84 Patentblatt 84/3**

(45) Bekanntmachung des Hinweises auf die Patenterteilung:
**06.11.85 Patentblatt 85/45**

(84) Benannte Vertragsstaaten:
**BE CH DE FR GB IT LI SE**

(56) Entgegenhaltungen:
**AT - B - 360 152**
**BE - A - 832 174**
**DE - A - 2 355 209**
**DE - A - 2 651 771**

**JOURNAL OF THE AMERICAN CHEMICAL SOCIETY, Vol. 101, 1979 Seiten 4403-4404 (S. UYEO et al.)**
**JOURNAL OF THE AMERICAN CHEMICAL SOCIETY, Vol. 96, 1974 Seiten 7582-7584 (L.D. CAMA et al.)**
**TETRAHEDRON LETTERS, Nr. 44, 1979 Seiten 4287-4290 (M. YOSHIOKA et al.)**
**TETRAHEDRON LETTERS, Nr. 44, 1979 Seiten 4327-4330 (T. AOKI et al.)**
**CANADIAN JOURNAL OF CHEMISTRY, Vol. 52, 1974 Seiten 3996-3999 (S. WOLFE et al.)**

(73) Patentinhaber: **BAYER AG, Konzernverwaltung RP Patentabteilung, D-5090 Leverkusen 1 Bayerwerk (DE)**

(72) Erfinder: **Hoppe, Dieter, Prof. Dr., Schulweg 19, D-3400 Göttingen (DE)**
Erfinder: **Kleemann, Heinz-Werner, Dr., Fiorilloweg 1, D-3400 Göttingen (DE)**

EP 0 098 478 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 7-Amino-1-dethia-1-oxa-3-hydroxy-methyl-cephem-4-carbonsäuren.

Es ist bereits bekannt geworden, dass 7-Amino-1-dethia-1-oxa-3-hydroxymethyl-cephem-4-carbonsäuren synthetisch aus 3-Azido-4-methylthio-azetidinon-1-α-phosphono-essigsäure durch Chlorierung, Substitution mit Monoacetyl-1,3-dihydroxyaceton und nachfolgende Wittig-Reaktion und Reduktion der Azidogruppe zugänglich ist (Merck, DOS 2355209, 6.11.1972; Cama und Christensen, J. Am. Chem. Soc., *96*, 7582 (1974); Yoshioka et al., Tetrahedron Letters *1979*, 4287; Uyeo et al., J. Am. Chem. Soc. *101*, 4403 (1979)), doch ist bei diesem Verfahren ein dreistufiger Aufbau des an das Azetidinonringsystem ankondensierten hydrierten Oxazinringsystems erforderlich, weil die Einführung des Ringfragments 1-3 in zwei Stufen am vorgefertigten Azetidinonring erfolgen muss, was angesichts der bekannten Empfindlichkeit der Azetidinone von Nachteil ist.

Ferner ist bereits bekannt geworden, dass 7-Amino-1-dethia-1-oxa-3-hydroxymethyl-cephem-4-carbonsäuren aus 3-Amino-azetidinon-4-chlor-1-essigsäuren oder 3,4-Oxazolinoazetidinon-essigsäuren zugänglich sind, die am α-C-Atom der Essigsäure einen Isopropenyl- oder Isopropylidenrest tragen und aus Penicillinen durch Ringöffnungs- und Entschwefelungsreaktionen zugänglich sind (Wolfe et al., Can. J. Chem. *52*, 3996 (1974); Belgische Patentschrift Nr. 832174; Aoki et al., Tetrahedron Letters, *1979*, 4327), doch ist bei diesem Verfahren eine Oxidation der Seitenkette am Azetidinonring bis zur erwünschten Hydroxymethylstufe (oder deren Oxidationsstufenäquivalent) notwendig und – da sie in Gegenwart des empfindlichen 3-Amino-substituierten Azetidinonrings durchgeführt werden muss – schwierig durchführbar.

Eine Zusammenfassung des Standes der Technik geben M. Narisada et al., J. Antibiotics, *35*, 463 (1982).

Es wurde nun gefunden, dass man 7-Amino-1-dethia-1-oxa-3-hydroxymethyl-cephem-4-carbonsäuren und ihre Derivate der allgemeinen Formel (I)

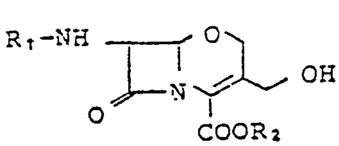
(I)

ihre Lactone der allgemeinen Formel (II)

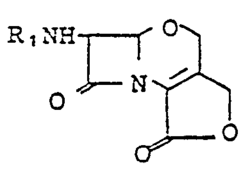
(II)

und ihre Doppelbindungsisomeren der allgemeinen Formel (III)

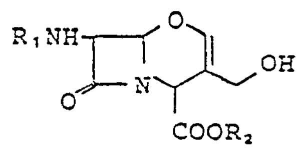
(III)

in denen

$R_1$ Wasserstoff oder ein in Cephalosporinen üblichen Acylrest einer organischen Carbonsäure, insbesondere mit bis zu 20 Kohlenstoffatomen oder die gegebenenfalls substituierte Benzyloxycarbonylgruppe ist, und

$R_2$ eine Carboxylschutzgruppe oder einen pharmazeutisch verwendbaren Esterrest bedeutet, dadurch erhält, dass man Verbindungen der allgemeinen Formel (IV)

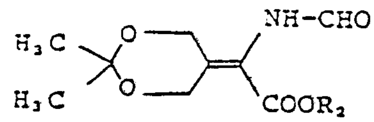
(IV)

in der

$R_2$ die oben angegebene Bedeutung hat, in einem Lösungsmittel, beispielsweise Dimethoxyethan, mit einem Schwefelungsmittel, beispielsweise Lawesson-Reagenz, zu Verbindungen der allgemeinen Formel (V)

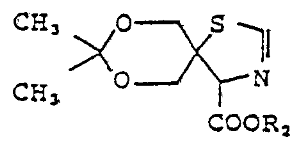
(V)

in der

$R_2$ die oben angegebene Bedeutung hat, umsetzt, diese anschliessend mit einer starken Base, beispielsweise Butyllithium, in einem Lösungsmittel, beispielsweise Tetrahydrofuran, bei niedrigen Temperaturen, beispielsweise bei −78° C und anschliessend mit einem Alkylierungsmittel, beispielsweise Methyljodid, zu Verbindungen der allgemeinen Formel (VI)

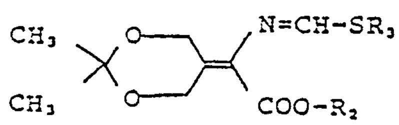
(VI)

oder deren Doppelbindungsisomeren der Formel (VI')

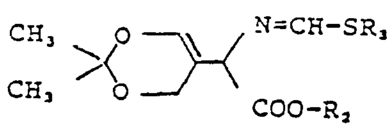
(VI')

in denen

$R_2$ die oben angegebene Bedeutung hat, und

$R_3$ gegebenenfalls substituiertes Alkyl bedeutet, umsetzt, oder indem man Verbindungen der allgemeinen Formel (IV') oder (IV'')

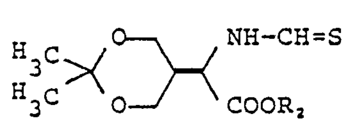
(IV')

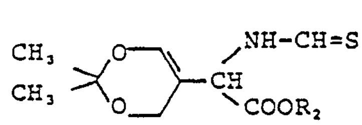
(IV'')

in denen

$R_2$ die oben angegebene Bedeutung hat, mit einem Alkylierungsmittel zu Verbindungen der allgemeinen Formel (VI) oder (VI') umsetzt, diese anschliessend mit einem aktivierten Derivat einer Säure beispielsweise dem Säurechlorid der allgemeinen Formel (VII)

$$X-CH_2-COOH \qquad (VII)$$

in der

X Azido oder Phthalimido bedeutet, in Gegenwart einer Base, beispielsweise Triethylamin, in einem Lösungsmittel, beispielsweise Methylenchlorid, zu Verbindungen der allgemeinen Formel (VIII)

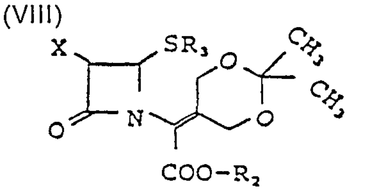

(VIII)

oder deren Doppelbindungsisomeren der Formel (VIII')

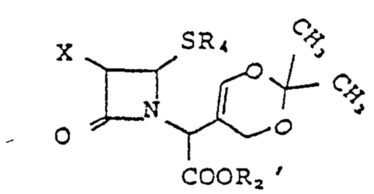

(VIII')

in denen

$R_2$, $R_3$ und X die oben angegebene Bedeutung haben, umsetzt, diese anschliessend im Falle, dass X Azido ist, mit einem Reduktionsmittel, beispielsweise Schwefelwasserstoff, in Gegenwart eines Amins, beispielsweise Triethylamin, in einem Lösungsmittel, beispielsweise Methylenchlorid oder im Falle, dass X Phthalimido ist, mit Phthalimid spaltenden Reagenzien, beispielsweise Hydrazin, zu Verbindungen der allgemeinen Formel (IX)

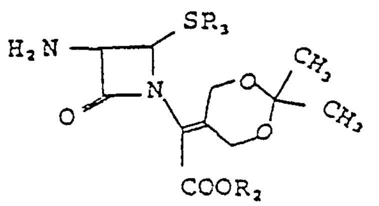

(IX)

oder deren Doppelbindungsisomeren der allgemeinen Formel (IX')

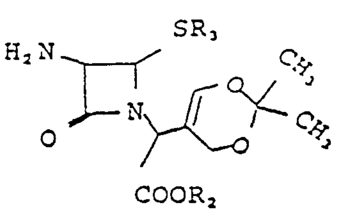

(IX')

in denen

$R_2$ und $R_3$ die oben angegebene Bedeutung haben, umsetzt, diese anschliessend mit einer an der Carboxylgruppe aktivierten Carbonsäure $R_1'-COOH$, bei der $R_1'$ einen organischen Rest bis zu 19 Kohlenstoffatomen bedeutet oder diese anschliessend mit Reagenzien wie z.B. Benzyloxycarbonylchlorid umsetzt, die die gegebenenfalls substituierte Benzyloxycarbonylgruppe einzuführen vermögen, zu Verbindungen der allgemeinen Formel (X)

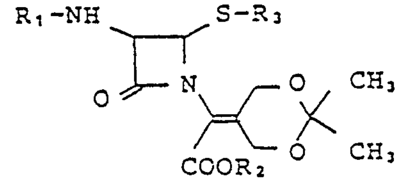

(X)

oder deren Doppelbindungsisomeren der allgemeinen Formel (X')

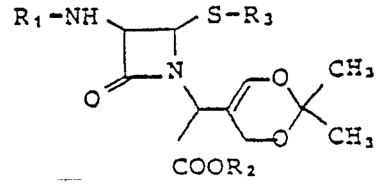

(X')

in denen

$R_1$, $R_2$ und $R_3$ die oben angegebene Bedeutung haben, umsetzt und die Verbindungen der allgemeinen Formel (X) anschliessend mit einem Chlorierungsmittel wie Chlor oder Sulfurylchlorid in einem gegen Chlorierung inerten Lösungsmittel wie Chloroform oder Essigester und anschliessend mit einer Protonensäure wie Trifluoressigsäure oder mit einer Lewissäure wie $BF_3$ im gleichen Lösungsmittel zu Verbindungen der allgemeinen Formel (I) oder (II) umsetzt und die Verbindungen der allgemeinen Formel (X') mit einem Chlorierungsmittel wie Chlor oder Sulfurylchlorid in einem gegen Chlorierung inerten Lösungsmittel wie Chloroform oder Essigester und anschliessend mit einer Protonensäure wie Trifluoressigsäure oder mit einer Lewissäure wie $BF_3$ zu Verbindungen der allgemeinen Formel (III)

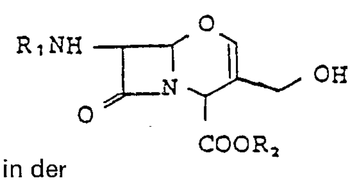

(III)

umsetzt, in der

$R_1$ und $R_2$ die oben angegebene Bedeutung haben.

Die Überführung der Verbindung der allgemeinen Formel (X) bzw. (X') in die Verbindungen der allgemeinen Formel (I), (II) bzw. (III) kann auch durch Umsetzung mit einem Chlorierungsmittel wie Chlor oder Sulfurylchlorid in einem gegen Chlorierung inerten Lösungsmittel wie Chloroform oder Essigester, anschliessende wässerige Aufarbeitung bei pH 7 zu Zwischenprodukten der allgemeinen Formel (XI) bzw. deren Doppelbindungsisomeren der allgemeinen Formel (XI')

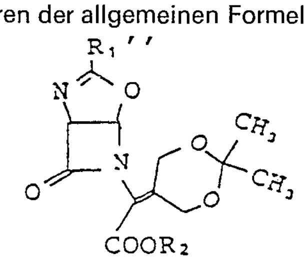

(XI)

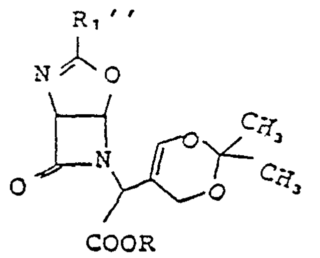

(XI')

in denen

$R_2$ die oben angegebene Bedeutung hat, und

$R_1''$ die Bedeutung von $R_1'$ hat und zusätzlich gegebenenfalls substituiertes Benzyloxy bedeutet, und deren weitere Umsetzung mit Protonensäuren wie Trifluoressigsäure oder Lewissäuren wie Bortrifluorid erfolgen.

Es ist als ausgesprochen überraschend zu bezeichnen, dass gemäss der erfindungsgemässen Umsetzungsfolge der Aufbau des 7-Amino-1-dethia-1-oxa-3-hydroxymethyl-cephem-4-carbonsäuresystems ohne Durchführung von Oxidationsreaktionen am intakten Azetidinonringsystem erfolgt und dass ein zweistufiger Aufbau des ankondensierten hydrierten Oxazinringsystems erfolgt, weil man erwarten musste, dass ein derartiger Aufbau nur unter Zuhilfenahme von Oxidationsreaktionen am intakten Azetidinonringsystem oder mittels eines mindestens dreistufigen Aufbaus des ankondensierten hydrierten Ringsystems erfolgen könne.

Das erfindungsgemässe Verfahren weist eine Reihe von Vorteilen auf. So kann beispielsweise dadurch, dass bereits mit Knüpfung des Azetidinonrings alle Gerüst- und Substituentenatome eingeführt sind und nur noch einfache Reduktions-, Substitutions- oder Eliminierungsreaktionen erforderlich sind, die überdies in ausgezeichneten Ausbeuten ablaufen, auf die Anknüpfung weiterer Gerüst- und Sustituentenatome am intakten Azetidinonring verzichtet werden, um die Verbindungen der allgemeinen Formeln (I), (II) und (III) zu erhalten.

Verwendet man die Verbindung (XII) als Ausgangsmaterial, so lässt sich der Reaktionsablauf durch das folgende Formelschema wiedergeben:

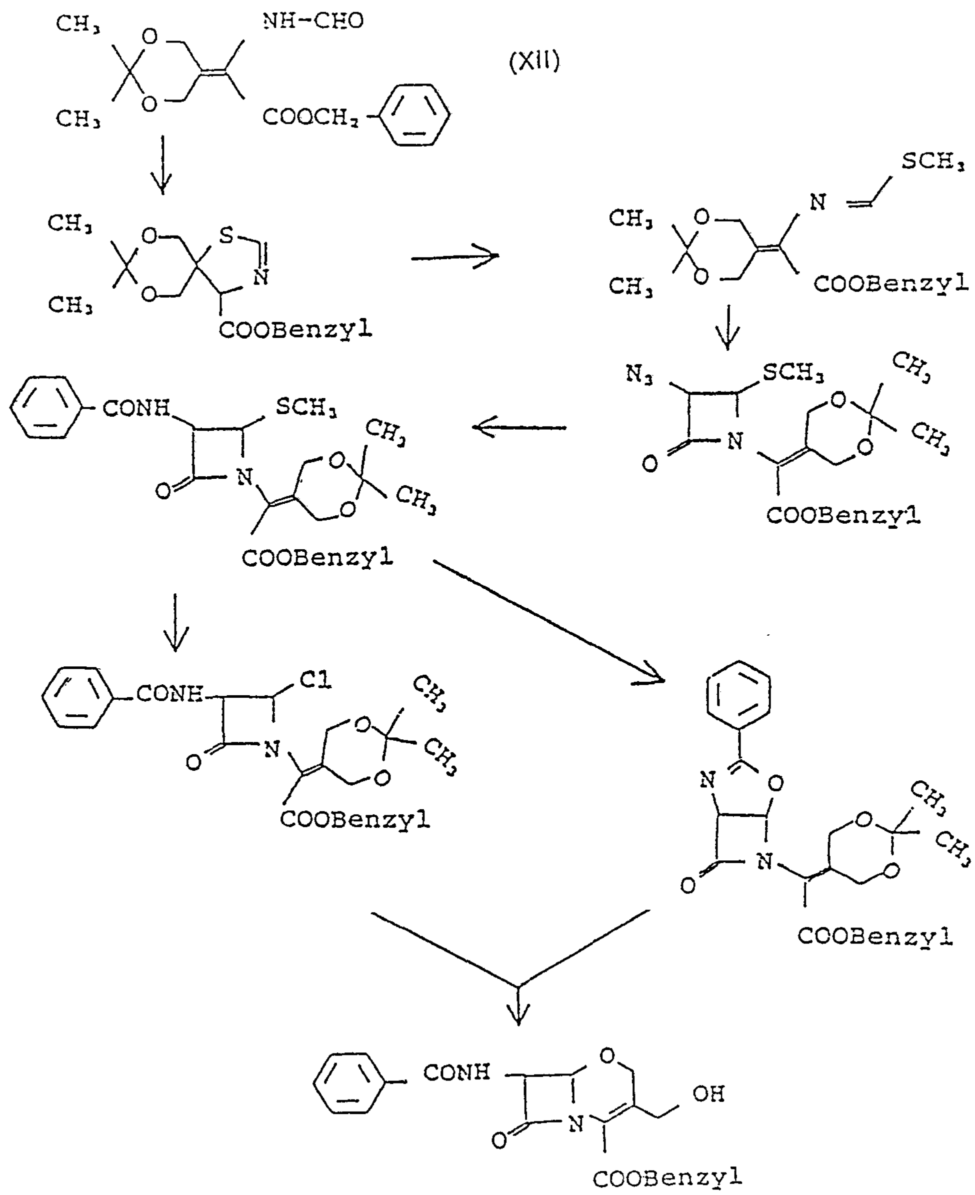

(XII)

In der allgemeinen Formel (IV) steht $R_2$ vorzugsweise für gegebenenfalls durch Methoxy oder Nitro substituiertes Benzyl oder Benzhydryl, für 1-Ethoxy-carbonyloxyethyl, Pivaloyloxymethyl, Acetoxymethyl, Phthalidyl, für β-Trimethylsilylethyl, β-Halogenethyl oder β-Trichlorethyl oder eine ähnliche, durch β-Eliminierung abspaltbare Schutzgruppe.

Die Verbindungen (IV) sind Acetonacetale.

Die Herstellung der Verbindungen (IV), (IV') und (IV'') ist in den Beispielen beschrieben oder in analoger Weise durchführbar. In den allgemeinen Formeln (VI) und (VI') bedeutet $R_3$ einen gegebenenfalls durch eine Estergruppe oder eine Carbonylgruppe substituierten Alkylrest mit bis zu 5 Kohlenstoffatomen. Da dieser Rest in der erfindungsgemässen Reaktionsfolge wieder abgespalten wird, können naturgemäss auch viele andere als die genannten Reste $R_3$ eingesetzt werden.

In den allgemeinen Formeln (X) und (X') bedeutet $R_1$ vorzugsweise die Benzyloxycarbonylgruppe oder den Rest $R_1'-CO-$ einer Carbonsäure, wobei $R_1'$ vorzugsweise den Resten (1) bis (17) entspricht, wobei die in den Resten vorkommenden Arylgruppen durch ein oder zwei Methoxy-, Hydroxy-, Amino- oder Aminomethylgruppen substituiert sein können, die ihrerseits durch Acetyl, Propionyl, Methoxycarbonyl, Ethoxycarbonyl oder – im Falle von Aminogruppen – Mesyl- oder Sulfogruppen acyliert sein können, und wobei aliphatische und heterocyclische Carboxyl- und Aminogruppen durch Schutzgruppen wie Benzyl, Benzhydryl, Trimethylsilylethyl bzw. Z-, BOC-, Trityl oder Cyanethoxycarbonyl geschützt vorliegen sollten.

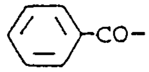
(1)

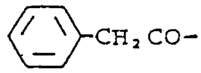
(2)

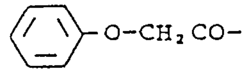
(3)

$NC-CH_2CO-$
(4)

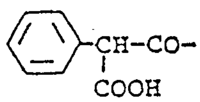
(5)

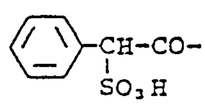
(6)

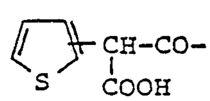
(7)

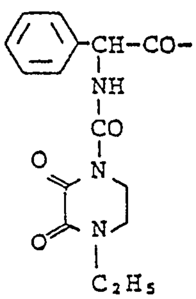
(8)

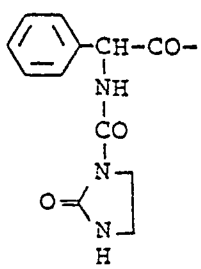
(9)

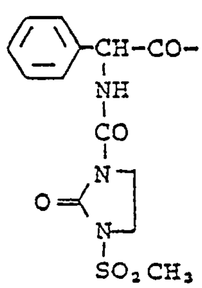
(10)

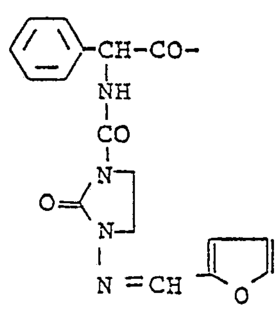
(11)

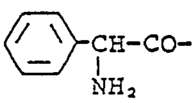
(12)

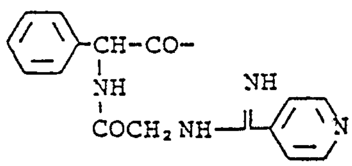
(13)

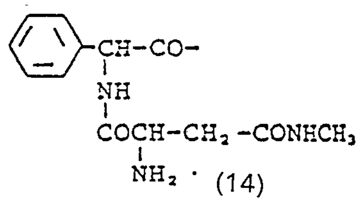
(14)

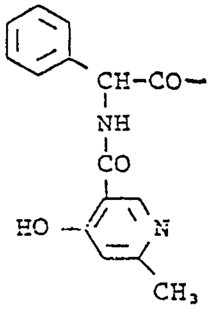
(15)

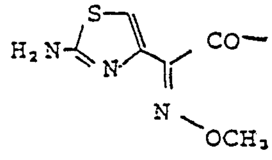
(16)

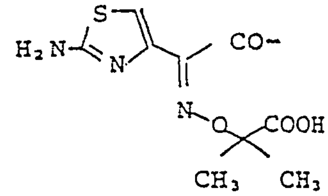
(17)

Bei der erfindungsgemässen Herstellung der Verbindungen der allgemeinen Formel (V) aus den Verbindungen der allgemeinen Formel (IV) kommen als Verdünnungsmittel beispielsweise Dimethoxyethan, Diglyme, Triglyme, Tetrahydrofuran, Dioxan, Diethylether, t-Butylmethylether, Dichlormethan, Tetrachlorkohlenstoff, 1,2-Dichlorethan, 1,1,2-Trichlorethan, Trichlorethylen, Chlorbenzol, Dichlorbenzol, Chloroform, Ethylacetat, Anilin, Piperidin, Toluol, Cyclohexan, Acetonitril, Nitromethan, als Schwefelungsmittel beispielsweise 2,4-Bis(4-methoxyphenyl)-2,4-dithioxo-1,3,2,4-dithiadi-phosphetan (Lawesson-Reagenz), 2,4-Bis(4-chlorphenyl)-2,4-dithioxo-1,3,2,4-dithiadiphosphetan, 2,4-Diphenyl-2,4-dithioxo-1,3,2,4-dithiadiphosphetan, Phosphorpentasulfid, Phosphortrisulfid, Dischwefeltetranitrid, Tetraschwefeltetranitrid, Trithiazylchlorid, elementarer Schwefel, Polyschwefeldichlorid in Frage.

Bei der erfindungsgemässen Herstellung der Verbindungen der allgemeinen Formel (VI) bzw. (VI') aus den Verbindungen der allgemeinen Formel (V) kommen als starke Basen ausser Butyllithium beispielsweise Phenyllithium, Lithiumdiisopropylamid, Lithiumhexamethyldisilazanid, Kalium-tert.-butylat, Natriumamid, Dimethylsulfoxid-Kalium, Lithiumhydroxid, Natriumethanolat, Natriumcarbonat, Kaliumcarbonat, als Verdünnungsmittel ausser Tetrahydrofuran beispielsweise Dioxan, Dimethoxyethan, Ammoniak, Dimethylsulfoxid, Methanol, Hexamethylphosphorsäuretriamid, Dimethylformamid, als Alkylierungsmittel ausser Methyljodid beispielsweise Dimethylsulfat, Methylsulfonat, Methyltrifluormethansulfonat, Trimethyloxonium-tetrafluoroborat, Allylbromid, Benzylbromid, Isopropyliodid, in Frage. Die gleichen Alkylierungsmittel kommen bei der erfindungsgemässen Umsetzung der Verbindungen der allgemeinen Formel (IV') bzw. (IV'') zu den Verbindungen der allgemeinen Formel (VI) bzw. (VI') in Frage.

Die Umsetzung wird bei Temperaturen von −78°C bis +40°C durchgeführt.

Bei der erfindungsgemässen Herstellung der Verbindungen der allgemeinen Formel (VIII) bzw. (VIII') aus Verbindungen der allgemeinen Formel (VI) bzw. (VI') kommen als Verdünnungsmittel ausser Dichlormethan beispielsweise Dichlorethan, Chlorbenzol, Toluol, Chloroform, Ethylacetat, Tetrahydrofuran, Dioxan, Diethylether, als Basen ausser Triethylamin beispielsweise Piperidin, N-Methylmorpholin, Picolon, Lutidin, Kaliumcarbonat, NaOH und Phasentransfer-Reagenzien wie beispielsweise Kronenether in Frage.

Die Umsetzung wird bei Temperaturen von −40 bis +60°C durchgeführt.

Bei der erfindungsgemässen Herstellung der Verbindungen der allgemeinen Formel (IX) bzw. (IX') aus Verbindungen der allgemeinen Formel (VIII) bzw. (VIII'), in denen X Azido bedeutet, kommen als Reduktionsmittel neben Schwefelwasserstoff/Pyridin beispielsweise Wasserstoff-Palladium-Kohle, Wasserstoff-Platin, Zink-Eisessig, Zinn-(II)-chlorid-Eisessig, wenn X Phthalimid bedeutet als imidspaltende Reagenzien neben Hydrazin beispielsweise substituierte Hydrazine und als Verdünnungsmittel beispielsweise Wasser, Dichlormethan, Dimethylformamid, Chlorbenzol, Toluol, Ethylacetat, Tetrahydrofuran, Ethanol, Eisessig in Frage.

Die Umsetzung wird bei Temperaturen von −30°C bis +90°C durchgeführt.

Bei der erfindungsgemässen Herstellung der Verbindungen der allgemeinen Formel (X) bzw. (X') aus Verbindungen der allgemeinen Formel (IX) kommen als Verdünnungsmittel beispielsweise Wasser, Acetonitril, Dichlormethan, Dichlorethan, Chlorbenzol, Tetrahydrofuran, Dimethylformamid, Hexamethylphosphorsäuretriamid, Dimethylsulfoxid, Nitrobenzol, Nitromethan, Sulfolan und im Falle, dass Basen wegen der Freisetzung von Säuren aus der aktivierten Carbonsäure $R_1'-COOH$ oder dem Benzyloxycarbonylgruppen einführenden Reagenz zugegeben werden, kommen als Basen beispielsweise Natronlauge, Natriumbicarbonat, Pyridin, Piperidin, Picolin, N-Methylmorpholin, Diisopropylamin, Triethylamin, 4-Dimethylaminopyridin, Polyvinylpyridin, oder basische Ionenaustauscher in Frage.

Die Umsetzung wird bei Temperaturen von −80°C bis +60°C durchgeführt. Als an der Carboxylgruppe aktivierte Carbonsäuren R'−COOH kommen bei dieser Umsetzung in geschützter oder ungeschützter Form beispielsweise Säuren der folgenden Formeln in Frage:

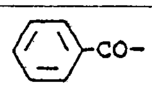 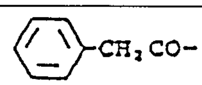 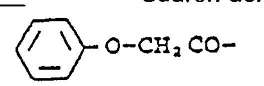 $NC-CH_2CO-$

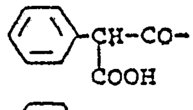 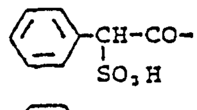 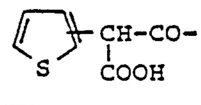

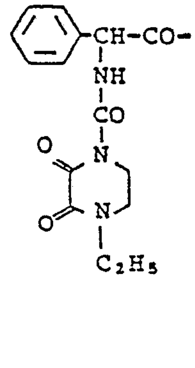 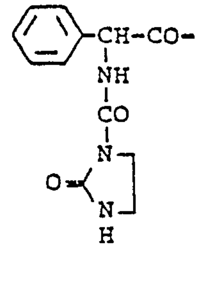 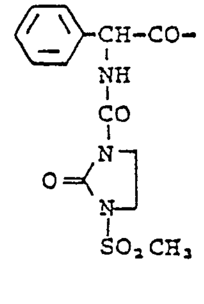 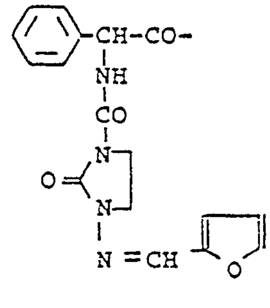

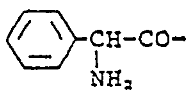

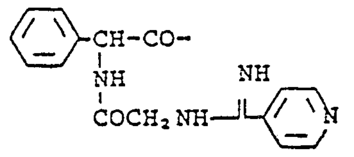

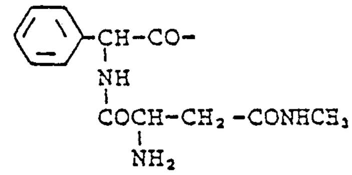

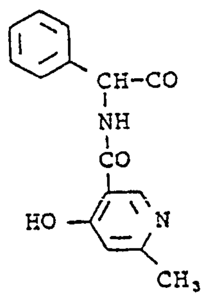

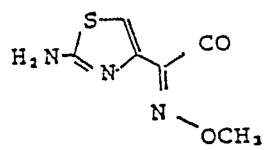

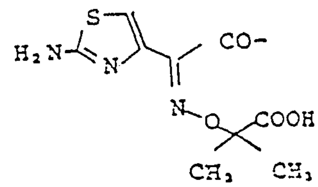

Zur Aktivierung der Carbonsäuren R'-COOH kommen alle in der Peptid- oder β-Lactamantibiotikachemie gebräuchlichen Aktivierungsmethoden wie beispielsweise die Säurechlorid-, Carbodiimid-, gemischte Anhydrid- oder aktivierte Estermethode in Frage.

Bei der erfindungsgemässen Herstellung der Verbindungen der allgemeinen Formel (I), (II) oder (III) aus Verbindungen der allgemeinen Formel (X) kommen als Chlorierungsmittel neben Chlor oder Sulfurylchlorid beispielsweise N-Chlorsuccinimid, Trichlorisocyanursäure, Chlorsulfonsäure in einem gegen Chlorisierung inerten Verdünnungsmittel wie beispielsweise Dichlorbenzol, Eisessig, Nitrobenzol, Tetrachlorkohlenstoff, Chloroform, Essigester, Dichlormethan, tert.-Butyl-methylether in Frage. Der Chlorierungsschritt wird bei Temperaturen von −80 bis +100°C durchgeführt. Zur Cyclisierung zu den Verbindungen der allgemeinen Formeln (I) und (II) aus den chlorierten Verbindungen der allgemeinen Formel (X) und zu (III) aus (X') kommen als Protonensäuren beispielsweise Trifluoressigsäure, Trifluormethansulfonsäure, Perchlorsäure, Tetrafluorborwasserstoffsäure, Chlorwasserstoffsäure, Quadratsäure, p-Toluolsulfonsäure, Campfersulfonsäure, Polyphosphorsäure, als Lewissäuren beispielsweise Bortrifluorid, Zink(II)chlorid, Aluminiumchlorid, Zinn(IV)-chlorid, Quecksilber(II)chlorid, Siliciumtetrachlorid, Trimethylsilyltrifluormethansulfonat, Trimethylsilyltrifluoracetat, als Verdünnungsmittel beispielsweise Die Verdünnungsmittel des Chlorierungsschrittes in Frage. Bei der erfindungsgemässen Herstellung der Verbindungen der allgemeinen Formel (XI) bzw. (XI') aus den Chlorierungsprodukten der Verbindungen der allgemeinen Formel (X) bzw. (X') kommt anstelle der wässerigen Aufarbeitung bei etwa pH 7 beispielsweise die Aufarbeitung mit Natriumhydrogencarbonat-Lösung, Dinatriumhydrogenphosphat-Lösung oder der Kontakt zu Kieselgel bzw. basischem Aluminiumoxid in Frage.

Eine Umwandlung der Produkte der allgemeinen Formeln (I), (II) und (III) ineinander und in andere bekannte 7-Amino-1-dethia-1-oxa-3-methyl-cephem-4-carbonsäuren, die an der Methylgruppe durchandere Reste als die OH-Gruppe substituiert sind, ist bei M. Narisada et al., J. Antibiotics, *35*, 463 (1982) zusammengefasst dargestellt.

*Beispiel 1:*

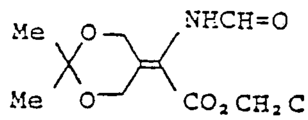

*2-(2,2-Dimethyl-1,3-dioxan-5-yliden)-2-(N-formyl)amino-essigsäure-benzylester*

Zu 10,0 g (80 mmol) 90% Kalium-tert.-butanolat in 50 ml wasserfreiem THF unter $N_2$ bei −60°C tropfte man 14,0 g (80 mmol) Isocyanessigsäure-benzylester (gelöst in 60 ml THF), rührte 10 min bei dieser Temperatur und tropfte 10,4 g (80 mmol) 2,2-Dimethyl-1,3-dioxan-5-on (in 40 ml THF) ein. Man setzte das Rühren fort (30 min bei −60°C und 30 min bei 0°C) und neutralisierte die Reaktionsmischung mit 4,8 g (80 mmol) Essigsäure in 200 ml Dichlormethan. Die Mischung wurde mit Wasser gewaschen (3×100 ml) über Natriumsulfat getrocknet und das Solvens im Vakuum entfernt. Man erhielt 19,8 g (81%) Produkt vom Schmo. 80°C.

IR (KBr): 333 (NH), 1743 (OC=O), 1698, 1671, 1517 (NHC=O) 1646 $cm^{-1}$ (C=C).

$^1$H-NMR ($CDCl_3$, δ): 1,31 (s, 2 $CH_3$), 4,16 und 4,38 (je s, $OCH_2$), 5,13 (s) und 7,3 (m) ($CH_2C_6H_5$), 7,60 und 8,06 (je br. s., NHCH=O).

*Beispiel 2:*

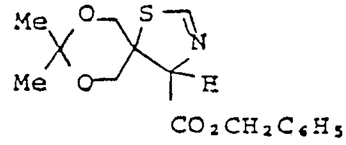

*2,2-Dimethyl-spiro[1,3-dioxan-5,5'-1',3'-thiazolin]-4'-carbonsäure-benzylester*

3,66 g (12,0 mmol) 2-(2,2-Dimethyl-1,3-dioxan-5-yliden)- 2 -(formylamino)essigsäurebenzylester (Beispiel 1) in 37 ml wasser- und peroxidfreiem 1,2-Dimethoxyethan wurden mit 2,91 g (7,2 mmol) 2,4-Bis-(4-methoxyphenyl)-2,4-dithioxo-1,3,2,4-dithiadiphosphetan (Lawesson-Reagenz) 20 min bei 20°C gerührt (DC-Kontrolle).

Es wurde festgestellt, dass die Reaktion mit einigen Chargen von handelsüblichen oder selbsthergestelltem Lawesson-Reagenz nicht ansprang. Es liess sich in diesen Fällen «aktivieren», indem man es kurz unter Feuchtigkeitsausschluss mit dem 30fachen Volumen 1,2-Dimethoxyethan (auf −20°C gekühlt) ausspülte.

Man rührte das Reaktionsgemisch in 120 ml gesättigte wässerige Natriumbicarbonat-Lösung ein und extrahierte die Lösung dreimal mit je 50 ml tert.-Butylmethylether. Trocknung der organischen Phase und Entfernung des Solvens im Vakuum ergab 3,76 g (98%) hellgelbes Öl.

IR (KBr-Pressling): 1743 (C=O), 1580 $cm^{-1}$ (C=N).

$^1H$-NMR ($CDCl_3$): δ=1,27 und 1,38 (je s; 2 $CH_3$), 3,78, 3,87, 3,95 und 4,08 (AB-Teile; 2 $CH_2$), 4,73 (d, J=1,8 Hz; C−4−H), 5,24 (s; $CH_2Ph$), 7,37 (s; $C_6H_5$), 8,07 (d, J=1,8 Hz; C−2−H).

*Beispiel 3:*

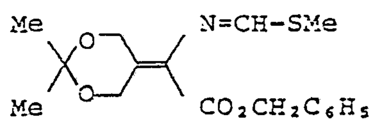

*2-(2,2-Dimethyl-1,3-dioxan-5-yliden)-2-[N-(methylthiomethylen)amino]essigsäure-benzylester*

230 mg (0,72 mmol) des ungereinigten Thiazolins (aus Beispiel 2), gelöst in 4 ml wasserfreiem Tetrahydrofuran unter $N_2$, wurden bei −78°C mit 0,50 ml einer 1,55 n Lösung (0,77 mmol) n-Butyllithium in Hexan versetzt und 15 min bei dieser Temperatur gerührt. Dann fügte man 0,47 ml (0,75 mmol) Methyliodid hinzu und rührte 1 h bei −30°C und 3 h bei 0°C. Das Reaktionsgemisch wurde durch eine Glasfritte, die mit 1 cm Kieselgur bedeckt war, filtriert. Aus dem Filtrat gewann man nach Abziehen des Solvens im Vakuum 150 mg (62%) dunkles Öl.

IR (KBr-Pressling): 1713 (C=O), 1588 $cm^{-1}$ (C=N).

$^1H$-NMR ($CDCl_3$): δ=1,41 (s; 2 $CH_3$), 2,40 (s; $SCH_3$), 4,53 und 4,67 (je s, 2 $CH_2$), 5,22 (s; $CH_2Ph$), 7,36 (s; $C_6H_5$), 8,56 (s; N=C−H).

*Beispiel 4:*

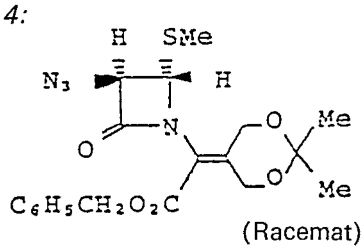

(Racemat)

*2-(trans-3-Azido-4-methylthio-2-oxo-1-azetidinyl)-2-(2,2-dimethyl-1,3-dioxan-5-yliden)essigsäure-benzylester*

Zur Lösung von 1,29 g (3,85 mmol) ungereinigtem Produkt von Beispiel 3 und von 0,80 ml (5,77 mmol) Triethylamin in 90 ml wasserfreiem Dichlormethan tropfte man unter Rühren bei 20°C binnen 90 min, 0,68 g (5,77 mmol) Azidoacetylchlorid in 10 ml Dichlormethan. Man rührte 30 min nach, zog das Solvens bei Raumtemperatur im Vakuum ab und nahm den Rückstand mit 90 ml Toluol auf, filtrierte von den ausgefallenen Salzen ab, zog das Solvens im Vakuum ab und chromatographierte den Rückstand an 50 g Kieselgel mit Diethylether/Petrolether (1:1), $R_f$=0,35. Man erhielt 1,35 g (84%) Produkt vom Schmp. 74°C (aus Petrolether).

| | | |
|---|---|---|
| *Analyse* für $C_{19}H_{22}N_4O_5S$ (418,47): | | |
| Berechnet: | C 54,53 | H 5,30% |
| Gefunden: | C 54,70 | H 5,38% |

IR (KBr): 2100 ($N_3$), 1775 (C=O–Lactam), 1718 (C=O–Ester), 1625 und 1640 $cm^{-1}$ (C=O).

$^1H$-NMR ($CDCl_3$): δ=1,40 (s; 2 $CH_3$), 1,98 (s; $SCH_3$), 4,18 (AB-Teil, $J_{AB}$=16,0 Hz; $CH_2O$ trans zur Carboxylgruppe), 4,44 (d, J=2,3 Hz; C−=−H), 4,48 (AB-Teil, d, $J_{AB}$=16,0 Hz, J=1,7 Hz; $CH_2O$ trans zur Estergruppe), 4,73 (AB-Teil, d, J=1,7 Hz; $CH_2O$ cis zur Estergruppe), 4,78 (AB-Teil, s; $CH_2O$ cis zur Estergruppe), 4,98 (d, J=2,3 Hz; C−4−H), 5,12 und 5,36 (AB, $J_{AB}$=11,8 Hz; $CH_2$–Benzyl), 7,37 (s; $C_6H_5$).

*Beispiel 5:*

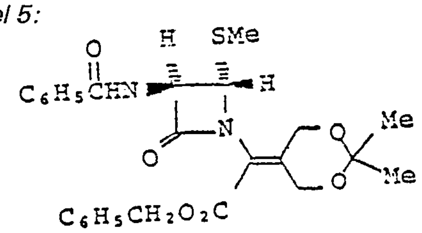

*2-(trans-3-Benzoylamino-4-methylthio-1-azetidinyl)-2-(2,2-dimethyl-1,3-dioxan-5-yliden)essigsäure-benzylester*

Zur Lösung von 0,64 g (1,53 mmol) Produkt von Beispiel 4 und 0,32 ml Triethylamin in 16 ml Dichlormethan leitete man bei 0°C so lange Schwefelwasserstoff, bis im IR-Spektrum einer Probe kein Azid (2100 $cm^{-1}$) mehr nachweisbar war (1,5 h). Man entfernte das Solvens im Vakuum, nahm den Rückstand in 65 ml wasserfreiem Chloroform auf, fügte 0,26 ml (3,2 mmol) Pyridin hinzu und tropfte dann bei −5°C eine Chloroform-Lösung von 0,38 ml (0,32 mmol) Benzoylchlorid ein. Die Reaktionsmischung wurde 1 h bei 20°C gerührt, dann dreimal mit je 30 ml Phosphatpuffer (pH 7) ausgeschüttelt, über Natriumsulfat getrocknet und dann das Solvens im Vakuum entfernt.

Die Chromatographie des Rückstands an Kieselgel (50 g) mit tert.-Butylmethylether/Petrolether (1:1) ergab 0,61 g (80%) Produkt vom Schmp. 67°C (aus tert.-Butylmethylether).

IR (KBr): 3360 (NH), 1773 (C=O−Lactam), 1720 (C=O−Ester), 1668 und 1535 $cm^{-1}$ (C=O−Amid).

$^1H$-NMR ($CDCl_3$): δ=1,42 (s; 2 $CH_3$), 2,07 (s; $SCH_3$), 4,26 und 4,55 (AB, $J_{AB}$=16,5 Hz; $CH_2O$), 4,66 und 4,90 (AB, $J_{AB}$=17,5 Hz; $CH_2O$), 5,15 (dd, $J_1$=7,7 Hz; $J_2$=2,5 Hz, C−=−H), 5,24 (d, J=2,5 Hz; C−4−H), 5,18 und 5,35 (AB,

$J_{AB}$=11,8 Hz; $CH_2$–Benzyl), 7,30-7,00 (m; NH, Aromaten–H).

$^{13}$C–NMR ($CDCl_3$): δ=12,4 ($SCH_3$), 23,7 und 23,9 (2 $CH_3$), 61,0 und 61,3 (2 $CH_2O$), 62,4 (C–3), 68,0 ($CH_2$–Benzyl), 68,2 (C–4), 100,8 (Acetonid–C–2), 115,7 (=C), 123,7, 128,8, 129,0, 132,3, 133,1 und 135,3 (Aromaten–C), 155,2 (N–C=), 162,6, 163,8 und 167,7 (C=O).

*Beispiel 6:*

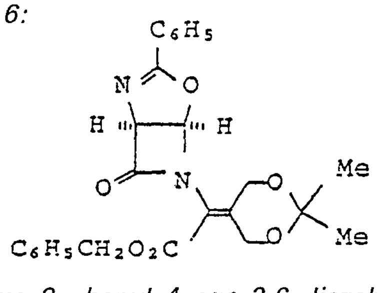

*2-(7-Oxo-3-phenyl-4-oxa-2,6-diazabicyclo-[3.2.0]hept-2-en-6-yl)-2-(2,2-dimethyl-1,3-dioxan-5-yliden)essigsäurebenzylester (5)*

Zu 106 mg (0,22 mmol) Produkt von Beispiel 5 in 5 ml trockenem Chloroform unter $N_2$ fügt man bei −40°C 0,75 ml (0,22 mmol) einer 0,3 M Lösung von Chlor in Tetrachlorkohlenstoff. Nach 15 min. erwärmte man auf −15°C, rührte noch 3 h bei dieser Temperatur und zog dann im Vakuum – zuletzt bei $10^{-4}$ Torr, das Solvens und die flüchtigen Nebenprodukte bei −15°C ab (ca. 4 h). Zum Rückstand gab man 6 ml (auf −15°C gekühltes) Aceton und dann 6 ml Phosphatpuffer (pH 7) und rührte die Mischung noch 1 h bei 20°C. Dann extrahierte man mit Dichlormethan (3× je 10 ml), trocknete die Extrakte über Natriumsulfat und Natriumhydrogencarbonat. Das nach dem Abziehen des Solvens im Vakuum zurückbleibende Rohprodukt wurde über Kieselgel (neutral, 8 g) mit tert.-Butylmethylether/Petrolether (1:1) chromatographiert. Man gewann 71 mg (72%) Produkt $R_f$=0,25, vom Schmp. 51°C (aus dem zur Chromatographie benutzten Gemisch).

IR (KBR): 1784 (C=O–Lactam), 1720 (C=O–Ester) und 1635 $cm^{-1}$ (C=N).

$^1$H–NMR ($CDCl_3$, δ): 1,30 (s, 2 $CH_3$), 3,88 und 4,30, 4,63 und 4,67 (je AB-Teile, je $OCH_2$), 5,02 und 5,23 (AB, J=12 Hz, $OCH_2PH$), 7,25-7,45 und 7,80-7,95 (m, $C_6H_5$), 5,33 und 6,18 (je d, J=3,5 Hz, β-Lactam-Protonen).

*Beispiel 7:*

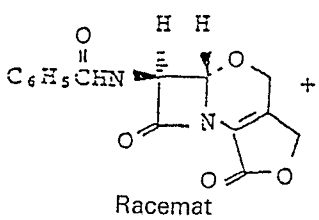

Racemat

+

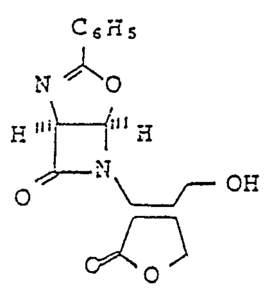

Racemat

*trans-7-Benzoylamino-3-hydroxymethyl-1-oxaceph-3-em-4-carbonsäure-butenolid* und *3-(7-Oxo-3-phenyl-4-oxa-2,6-diazabicyclo-(3.2.0)hept-2-en-6-yl)-4-hydroxymethyl-2,5-dihydrofuran-2-on*

24 mg (0,54 mmol) Produkt von Beispiel 6 wurden mit 0,4 ml Trifluoressigsäure unter $N_2$ 1 h bei −13°C gerührt Dann zog man das Solvens bei −10°C und $10^{-4}$ Torr ab (1 h), nahm den Rückstand mit 0,5 ml Dichlormethan auf und chromatographierte die Lösung an 8 g Kieselgel mit tert.-Butylmethylether. Man gewann 9 mg (56%) einer 4:6-Mischung von trans-7-Benzoylamino-3-hydroxymethyl-1-oxaceph-3-em-4-carbonsäure-Butenolid und 3-(7-Oxo-3-phenyl-4-oxa-2,6-diazabicyclo(3.2.0)hept-2-en-6-yl)-4-hydroxymethyl-2,5-dihydrofuran-2-on, diese liessen sich durch Chromatographie mit Toluol/tert.-Butylmethylether an Kieselgel (bei teilweiser Zersetzung) trennen.

trans-7-Benzoylamino-3-hydroxymethyl-1-oxaceph-3-em-4-carbonsäure-butenolid

IR (KBr): 1787 (C=O–Lactam), 1760 (C=O–Butenolid), 1658 und 1530 $cm^{-1}$ (C=O–Amid).

$^1$H–NMR ($CDCl_3$, δ): 5,05 (dd, J=2,0 und 7,5 Hz, C–7–H), 6,57 (d, J=2,0 Hz, C–6–H), 4,42 und 4,81 (AB, J=15 Hz, $OCH_2$–Butenolid), 4,93 (s, 2–$CH_2$), 7,03 (d, J=7,5 Hz, NH), 7,25-8,05 (m, Phenyl).

MS: $C_{15}H_{12}N_2O_5$ Ber. 300.0746 Gef. 300.0746

3-(7-Oxo-3-phenyl-4-oxa-2,6-diazabicyclo-(3.2.0)hept-2-en-6-yl)-4-hydroxymethyl-2,5-dihydrofuran-2-on:

IR (KBr): 1773 (C=O, Lactam), 1757 (C=O, Butenolid), 1673 (C=C), 1632 $cm^{-1}$ (C=N).

$^1$H–NMR ($CDCl_3$, δ): 2,81 (br, s, OH), 5,46 und 6,88 (je d, J=3,4 Hz, β-Lactam–H), 4,40 und 4,70 (AB, J=15 Hz, $OCH_2$-Butenolid), 4,90 und 4,92 (AB, $CH_2$–OH), 7,2-8,1 (m, Phenyl).

MS: $C_{15}H_{12}N_2O_5$ Ber. 300.0746 Gef. 300.0746

*Beispiel 8:*

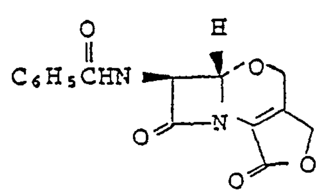

Racemat

*trans-7-Benzoylamino-3-hydroxymethyl-1-oxaceph-3-em-4-carbonsäure-butenolid 6* aus *4*

Zu 20 mg (0,04 mmol) Produkt von Beispiel 5 in 1,5 ml trockenem Chloroform unter $N_2$ fügte man 0,14 ml (0,04 mmol) einer 0,3 m Lösung von Chlor in Tetrachlormethan, erwärmte von −40°C auf −15°C und rührte 3 h bei dieser Temperatur. Man entfernte die flüchtigen Bestandteile im Vakuum (zuletzt bei $10^{-4}$ Torr, 4 h), nahm den Rückstand mit 0,5 ml trockenem Deutero-Chloroform auf und fügte 0,1 ml Trifluoressigsäure hinzu. Nach 10 min bei 20°C liessen sich $^1$H–NMR-spektroskopisch neben äquimolaren Mengen Aceton und Benzylalkohol nur Titelprodukt nachweisen.

*Beispiel 9:*

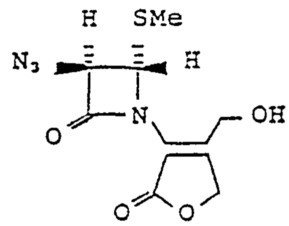

*3-(trans-3-Azido-4-methylthio-2-oxo-1-azetidinyl)-4-hydroxymethyl-2,5-dihydro-2-furanon*

59 mg (0,14 mmol) Produkt von Beispiel 4 in 2 ml trockenem Dichlormethan wurden mit 19 mg (0,14 mmol) wasserfreiem Zinkchlorid 12 h bei 20°C gerührt. Die Reaktionsmischung wurde an 10 g Kieselgel mit Diethylether chromatographiert und ergab 13 mg (34%) *8*, $R_f$=0,23, als zähes farbloses Öl.

$^1$H−NMR ($CDCl_3$, δ): 2,24 (s, $SCH_3$), 4,55 (AB, J=16,2 Hz, $OCH_2$), 4,65 und 4,94 (je d, J=2,6 Hz, C−3− und C−4−H).

IR ($CDCl_3$): 2103 ($N_3$), 1770 und 1764 (je C=O und 1675 cm$^{-1}$ (C=C).

*Beispiel 10:*

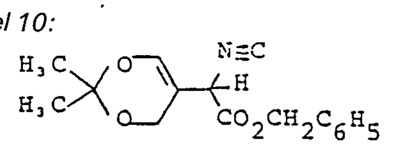

*2-(2,2-Dimethyl-4,5-dehydro-1,3-dioxan-5-yl)-2-isocyanoessigsäure-benzylester*

Zu 19,8 g (0,065 mol) 2-(2,2-Dimethyl-4,5-dehydro-1,3-dioxan-5-yl)-2-(N-formyl)aminoessigsäure-benzylester (Beispiel 1) und 18,2 g (0,180 mol) Triethylamin in 65 ml trockenem Dichlormethan wurden bei −15°C unter kräftigem Rühren 11,0 g (0,72 mol) Phosphoroxychlorid getropft. Das Gemisch wurde 2 h bei 20°C gerührt und dann bei 15-20°C mit 65 ml einer 20%igen wässrigen $K_2CO_3$-Lösung gerührt, bis keine $CO_2$-Entwicklung mehr beobachtet wurde. Nach Waschen der organischen Phase mit Wasser (2×65 ml) wurde sie getrocknet und das Solvens im Vakuum abgezogen. Umkristallisation des Rückstands aus Diethylether/Petrolether (1:1) ergab 11,0 g (61%) Produkt vom Schmp. 78°C.

IR ($CCl_4$): 2110 (NC), 1735 (C=O), 1660 cm$^{-1}$ (O−C=C−).

$^1$H−NMR ($CDCl_3$): δ=1,28 und 1,36 (je s; 2 $CH_3$), 3,96 und 4,26 (AB−Teil; $CH_2$), 4,68 (s; H), 5,13 (s; $CH_2$−Benzyl), 6,56 (s; Vinyl−H 7,23 (s; $C_6H_5$).

*Beispiel 11:*

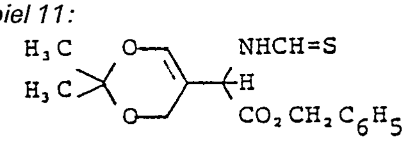

*2-(2,2-Dimethyl-4,5-dehydro-1,3-dioxan-5-yl)-2-(N-thioformyl)-aminoessigsäure-benzylester*

In 9,5 g (35 mmol) des Isocyanids aus Beispiel 10 und 2 ml Triethylamin in 50 ml Dichlormethan wurde bei 10-20°C Schwefelwasserstoff eingeleitet, bis im IR-Spektrum einer Probe kein Isocyanid (2100 cm$^{-1}$) mehr nachweisbar war (1-2 h). Nach Zufügen von 10 ml Toluol wurde das Solvens im Vakuum abgezogen, der Rückstand mit 200 ml Ether aufgenommen, mit 1 N $NaH_2PO_4$-Lösung und dann mit ges. KCl-Lösung gewaschen, die Lösung getrocknet. Nach dem Abziehen des Ethers im Vakuum blieben 10,3 g (100%) des Thioformamids zurück. Eine Analysenprobe wurde aus Diethylether/Petrolether (1:1) umkristallisiert (Schmp. 89°C).

IR (KBr-Pressling): 3290 (NH), 1715 (C=O), 1665 cm$^{-1}$ (=C).

$^1$H−NMR ($CCl_4$): δ=1,35 und 1,38 (je s; 2 $CH_3$), 4,11 (s, breit; O−$CH_2$), 5,20 und 5,23 (AB-Teil; $CH_2$−Benzyl), 5,52 (d, J=7,0 Hz; H−C−$CO_2$Bz), 6,56 (s, breit; O−CH=), 7,34 (s; $C_6H_5$), 8,2-8,4 (s, sehr breit; NH, tauscht mit $D_2O$ aus), 9,42 (d, J=6,0 Hz; CH=S).

*Analyse* für $C_{16}H_{19}NO_4S$ (321,39):

| | | | |
|---|---|---|---|
| Berechnet: | C 59,79 | H 5,96 | S 9,98% |
| Gefunden: | C 59,65 | H 6,03 | S 9,97% |

*Beispiel 12:*

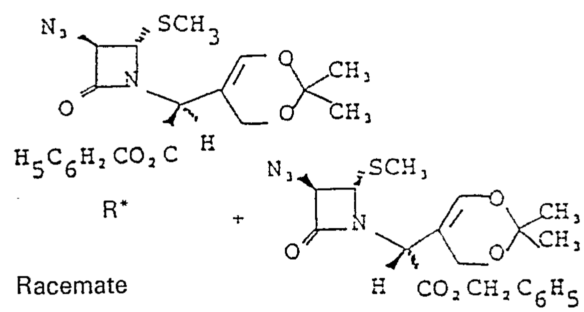

R*

+

Racemate

S*

*2-(trans-3-Azido-4-methylthio-2-oxo-1-azetidinyl)-2-(2,2-dimethyl-4,5-dehydro-1,3-dioxan-5-yl)essigsäure-benzylester*

Zu 0,8 g (25 mmol) des ungereinigten Thioformamids aus Beispiel 11 in 30 ml trockenem Tetrahydrofuran unter $N_2$ bei −78°C wurde eine Lösung von 3,0 g (25 mmol) von 90%igem Kalium-tert.-butanolat in 30 ml THF getropft. Nach 15 min wurden 3,75 g (26 mmol) Methyljodid hinzugefügt, die Mischung auf 0°C erwärmt und 1 h bei dieser Temperatur gerührt. Das ausgefallene Natriumjodid wurde unter $N_2$ über Kieselgur abfiltriert und das Solvens im Vakuum abgezogen. Das zurückbleibende gelbe Öl (8,4 g) wurde in 300 ml Dichlormethan (über $P_4O_{10}$ destilliert) gelöst, mit 2,0 g (33 mmol) Triethylamin in Dichlormethan versetzt und unter kräftigem Rühren bei 20°C binnen 5 h 3,35 g (28 mmol) Azidoacetylchlorid in 50 ml $CH_2Cl_2$ eingetropft. Anschliessend wurde 30 min nachgerührt, das Solvens im Vakuum abgezogen, der Rückstand mit 100 ml Benzol aufgenommen, das ausgefallene Hydrochlorid abgesaugt und der Rückstand im Vakuum vom Solvens befreit. Chromatographie über 1 kg Kieselgel mit Diethylether/Petrolether (1:1) ergab 5,0 g (48%) Produkt als 1:1-Mischung der Diastereomeren R* und S* als hellgelbes Öl (R*, $R_f$=0,41; S*, $R_f$=0,33).

IR ($CCl_4$): 2095 ($N_3$), 1765 (C=O−Lactam), 1735 (C=O−Ester), 1655 $cm^{-1}$ (C=C).

$^1H$−NMR ($CCl_4$:

*R** δ=1,37 (s; 2 $CH_3$), 1,93 (s; $SCH_3$), 4,14 (s, breit; Ring−$CH_2$), 4,24 (s; H−C−$CO_2$−Bz), 4,32 (d, J=2,0 Hz;C−3−H), 4,39 (d, J=2,0 Hz; C−4−H), 5,15 (s, breit; $CH_2$-Benzyl), 6,34 (s, breit; Ring−C=CH), 7,29 (s; $C_6H_5$).

*S** δ=1,37 (s; 2 $CH_3$), 2,01 (s; $SCH_3$), 4,04 und 4,14 (AB-Teil; Ring-$CH_2$), 4,24 (s; H−C−$CO_2$−Bz), 4,28 (d, J=2,0 Hz; C−4−H) −4,51 (d, J=2,0 Hz; C−3−H), 5,15 (s, breit; $CH_2$−Benzyl), 6,44 (s, breit; Ring−C=CH), 7,29 (s; $C_6H_5$).

*Beispiel 13:*

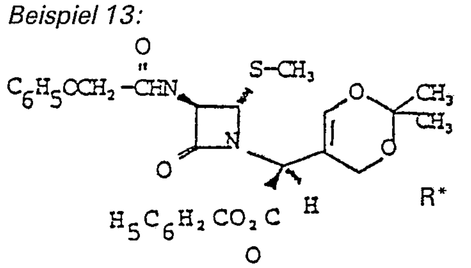

R*

+

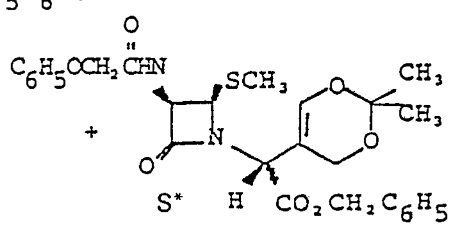

S*

*2-(trans-4-Methylthio-2-oxo-3-N-phenoxyacetylamino-1-azetidinyl)-2-(2,2-dimethyl-4,5-dehydro-1,3-dioxan-5-yl)essigsäure-benzylester*

In die Lösung von 2,44 g (5,8 mmol) des Azids aus Beispiel 12 und 0,96 ml (6,9 mmol) Triethylamin in 60 ml Dichlormethan wurde bei 0°C ein Schwefelwasserstoff-Strom geleitet, bis im IR-Spektrum einer Probe kein Azid (2100 $cm^{-1}$) mehr nachweisbar war (1-2 h). Das Solvens wurde im Vakuum abgezogen, der Rückstand in 20 ml trockenem Chloroform und 0,48 g (6,0 mmol) Pyridin gelöst und bei −5°C 1,03 g (6,0 mmol) Phenoxyacetylchlorid in 25 ml Chloroform hinzugetropft. Die Reaktionsmischung wurde 1 h bei 20°C gerührt, mit Phosphatpuffer (pH=7) ausgeschüttelt, über $MgSO_4$ getrocknet und das Lösungsmittel im Vakuum entfernt und der Rückstand über 300 g Kieselgel mit Essigsäureethylester/Cyclohexan (1:1) chromatographiert. Ausbeute 2,50 g (82%) gelbes Öl als 1:1-Mischung der Diastereomeren I ($R_F$=0,41) und B ($R_F$=0,36). Eine Probe des Diastereomeren B wurde durch Kristallisation aus Diethylether reingewonnen, Schmp. 51°C.

IR ($CCl_4$): 3200-3450 (NH), 1770 (C=O−Lactam), 1740 (C=O−Ester), 1690 (C=O−Amido), 1670 $cm^{-1}$ (C=C).

$^1H$−NMR ($CDCl_3$):

*R** δ=1,44 und 1,46 (je s; 2 $CH_3$), 2,02 (s; $SCH_3$), 4,35 und 4,40 (AB-Teil; Ring-$CH_2$), 4,48 (s; O−$CH_2$−C=O), 4,53 (s; HC−$CO_2$Bz), 4,77 (dd, $J_1$=2,3 Hz, $J_2$=8,7 Hz; C−3−H), 4,89 (d, J=2,3 Hz; C−4−H), 5,19 (s, breit; $CH_2$−Benzyl), 6,80-7,45 (m; NH, O−$C_6H_5$).

*S** δ=1,39 und 1,43 (je s; 2 $CH_3$), 2,04 (s; $SCH_3$), 4,23 und 4,39 (AB-Teil; Ring-$CH_2$), 4,46 (s; O−$CH_2$−C=O), 4,53 (s; HC−$CO_2$−Bz), 4,73 (d, J=2,3 Hz; C−4−H), 4,88 (dd, $J_1$=2,3 Hz, $J_2$=8,0 Hz; C−3−H), 5,22 und 5,23 (AB-Teil; $CH_2$−Benzyl), 6,55 (s, breit; Ring−C=C−H), 7,30 (s; $C_6H_5$−Benzyl), 6,80-7,45 (m; NH, O−$C_6H_5$).

*Analyse* für $C_{27}H_{30}N_2O_7S$ (526,61):

| | | |
|---|---|---|
| Berechnet: | C 61,58 | H 5,74% |
| Gefunden: | C 61,51 | H 5,76% |

## Patentansprüche

1. Verfahren zur Herstellung von 7-Amino-1-dethia-1-oxa-3-hydroxymethyl-cephem-4-carbonsäuren und ihrer Derivate der allgemeinen Formel (I)

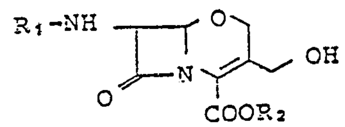

(I)

ihrer Lactone der allgemeinen Formel (II)

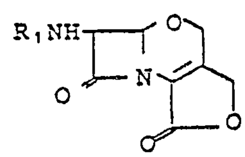

(II)

und ihrer Doppelbindungsisomeren der allgemeinen Formel (III)

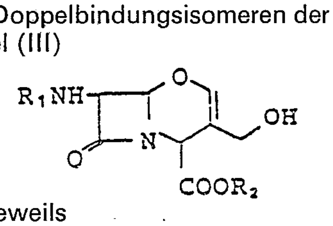

(III)

in denen jeweils

$R_1$ Wasserstoff oder ein in Cephalosporinen üblicher Acylrest einer organischen Carbonsäure oder die gegebenenfalls substituierte Benzyloxycarbonylgruppe ist und

$R_2$ eine Carboxylschutzgruppe oder einen pharmazeutisch verwendbaren Esterrest bedeutet, dadurch gekennzeichnet, dass man

a) eine Verbindung der allgemeinen Formel (IV)

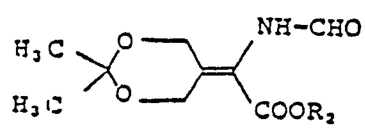

(IV)

in der

$R_2$ die oben angegebene Bedeutung hat, in einem Lösungsmittel mit einem Schwefelungsmittel zu Verbindungen der allgemeinen Formel (V)

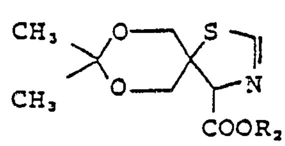

(V)

in der

$R_2$ die oben angegebene Bedeutung hat, umsetzt, diese anschliessend

b) mit einer starken Base in einem Lösungsmittel bei niedrigen Temperaturen, und anschliessend mit einem Alkylierungsmittel zu Verbindungen der allgemeinen Formel (VI)

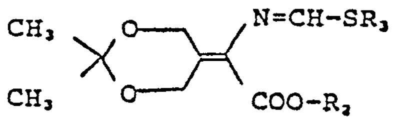

(VI)

oder deren Doppelbindungsisomeren der Formel (VI')

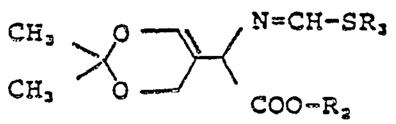

(VI')

umsetzt, wobei

$R_2$ die oben angegebene Bedeutung hat und

$R_3$ gegebenenfalls substituiertes Alkyl bedeutet, oder indem man Verbindungen der allgemeinen Formel (IV') oder (IV'')

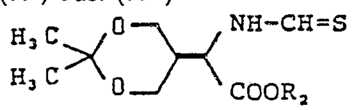

(IV')

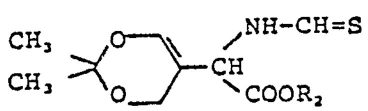

(IV'')

in denen

$R_2$ die oben angegebene Bedeutung hat, mit einem Alkylierungsmittel zu Verbindungen der allgemeinen Formel (VI) oder (VI') umsetzt, diese anschliessend

c) mit einem aktivierten Derivat einer Säure der allgemeinen Formel (VII)

$$X-CH_2-COOH \qquad (VII)$$

in der

X Azido oder Phthalimido bedeutet, in Gegenwart einer Base in einem Lösungsmittel zu Verbindungen der allgemeinen Formel (VIII)

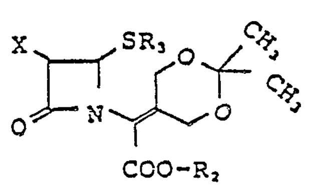

(VIII)

oder deren Doppelbindungsisomeren der Formel (VIII')

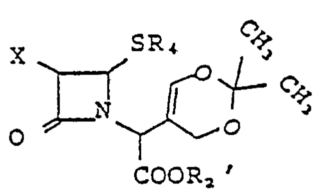

(VIII')

wobei

$R_2$, $R_3$ und X die oben angegebenen Bedeutungen haben, diese anschliessend

d) im Falle, dass X=Azido ist, mit einem Reduktionsmittel in Gegenwart eines Amins in einem Lösungsmittel, oder im Falle, dass X=Phthalimido ist, mit Phthalimid spaltenden Reagenzien zu Verbindungen der allgemeinen Formel (IX)

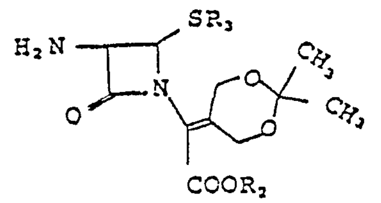

(IX)

oder deren Doppelbindungsisomeren der allgemeinen Formel (IX')

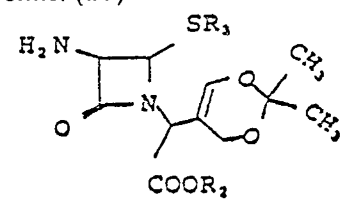

(IX')

umsetzt, wobei

$R_2$ und $R_3$ die oben angegebene Bedeutung haben, diese anschliessend

e) mit einer an der Carboxylgruppe aktivierten Carbonsäure $R_1'-COOH$, bei der $R_1'$ einen organischen Rest bedeutet oder diese anschliessend mit Reagenzien umsetzt, die die gegebenenfalls substituierte Benzyloxycarbonylgruppe einzuführen vermögen, zu Verbindungen der allgemeinen Formel (X)

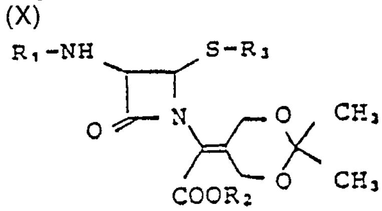

(X)

oder deren Doppelbindungsisomeren der allgemeinen Formel (X')

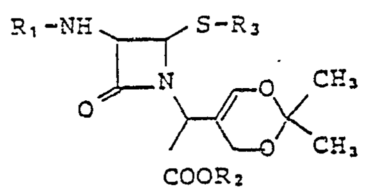

(X')

in denen

$R_1$, $R_2$ und $R_3$ die oben angegebenen Bedeutungen haben und

f) die Verbindungen der allgemeinen Formel (X) anschliessend mit einem Chlorierungsmittel in einen gegen Chlorierung inerten Lösungsmittel und anschliessend mit einer Protonensäure oder mit einer Lewissäure im gleichen Lösungsmittel zu Verbindungen der allgemeinen Formel (I) oder (II) umsetzt und die Verbindungen der allgemeinen Formel (X') mit einem Chlorierungsmittel in einem gegen Chlorierung inerten Lösungsmittel und anschliessend mit einer Protonensäure oder mit einer Lewissäure zu Verbindungen der allgemeinen Formel (III) umsetzt.

2. Verfahren nach Anspruch 1, dadurch gekennzeichnet, dass bei der Umsetzung der Verbindung (IV) zur Verbindung (V) das Schwefelungsmittel Lawesson-Reagenz ist.

3. Verfahren nach den Ansprüchen 1 oder 2, dadurch gekennzeichnet, dass die starke Base im Reaktionsschritt b) Butyllithium ist.

4. Verfahren nach den Ansprüchen 1 bis 3, dadurch gekennzeichnet, dass das Alkylierungsmittel im Reaktionsschritt b) Methyljodid ist.

5. Verfahren nach den Ansprüchen 1 bis 4, dadurch gekennzeichnet, dass das Chlorierungsmittel im Reaktionsschritt f) Chlor oder Sulfurylchlorid ist.

## Claims

1. Process for the preparation of 7-amino-1-dethia-1-oxa-3-hydroxymethyl-cephem-4-carboxylic acids and their derivatives of the general formula (I)

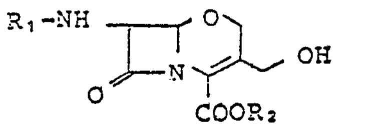

(I)

their lactones of the general formula (II)

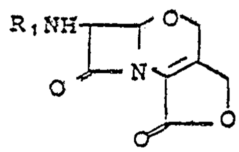

(II)

and their double bond isomers of the general formula (III)

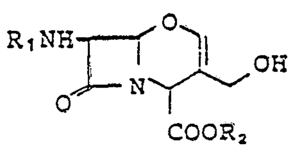

(III)

in each of which

$R_1$ is hydrogen or an acyl radical of an organic carboxylic acid customary in cephalosporinis or the optionally substituted benzyloxycarbonyl group and

$R_2$ denotes a carboxyl protective group or a pharmaceutically usable ester radical, characterised in that

a) a compound of the general formula (IV)

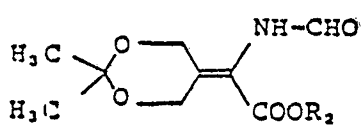

(IV)

in which

$R_2$ has the abovementioned meaning, is reacted in a solvent with a sulphurisation agent to give compounds of the general formula (V)

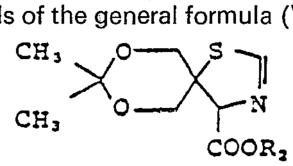

(V)

in which

$R_2$ has the abovementioned meaning, these are then

b) reacted with a strong base in a solvent at low temperatures, and then with an alkylating agent to give compounds of the general formula (VI)

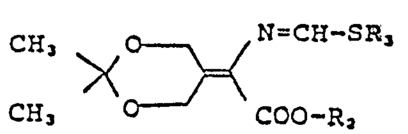

(VI)

or their double bond isomers of the formula (VI')

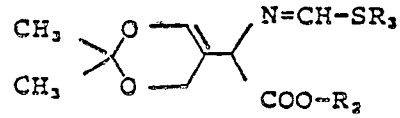

(VI')

wherein

$R_2$ has the abovementioned meaning and

$R_3$ denotes optionally substituted alkyl, or by reacting compounds of the general formula (IV') or (IV")

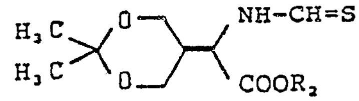

(IV')

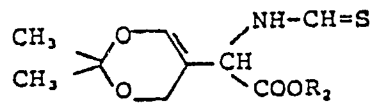

(IV")

in which

$R_2$ has the abovementioned meaning, with an alkylating agent to give compounds of the general formula (VI) or (VI'), these are then

c) reacted with an activated derivative of an acid of the general formula (VII)

$$X-CH_2-COOH \qquad (VII)$$

in which

X denotes azido or phthalimido, in the presence of a base in a solvent to give compounds of the general formula (VIII)

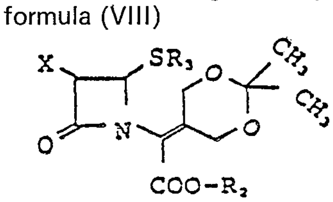

(VIII)

or their double bond isomers of the formula (VIII')

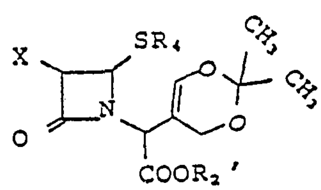

(VIII')

wherein

$R_2$, $R_3$ and X have the abovementioned meanings, these are then

d) in the case where X=azido, reacted with a reducing agent in the presence of an amine in a solvent, or in the case where X=phthalimido, with phthalimide-cleaving reagents, to give compounds of the general formula (IX)

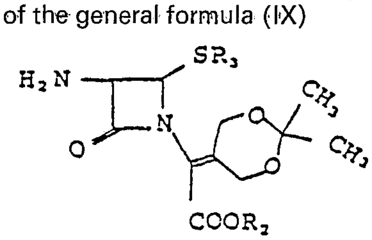

(IX)

or their double bond isomers of the general formula (IX')

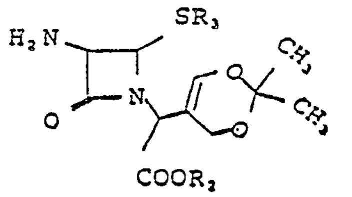

(IX')

wherein

$R_2$ and $R_3$ have the abovementioned meaning, these are then

e) reacted with a carboxylic acid $R_1'-COOH$ activated at the carboxylic group, in which $R_1'$ denotes an organic radical, or these are then reacted with reagents which are capable of introducing the optionally substituted benzyloxycarbonyl group, to give compounds of the general formula (X)

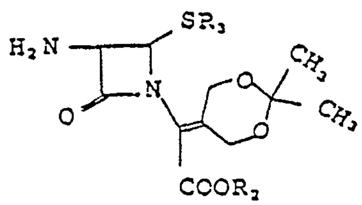

(X)

or their double bond isomers of the general formula (X')

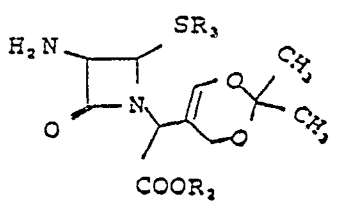

(X')

in which

$R_1$, $R_2$ and $R_3$ have the abovementioned meanings and

f) the compounds of the general formula (X) are then reacted with a chlorinating agent in a solvent which is inert to chlorination and then with a protonic acid or with a Lewis acid in the same solvent to give compounds of the general formula (I) or (II) and the compounds of the general formula (X') are reacted with a chlorinating agent in a solvent which is inert to chlorination and then with a protonic acid or with a Lewis acid to give compounds of the general formula (III).

2. Process according to Claim 1, characterised in that in the conversion of the compound (IV) to the compound (V) the sulphurisation agent is Lawesson reagent.

3. Process according to Claim 1 or 2, characterised in that the strong base in reaction step b) is butyl lithium.

4. Process according to Claims 1 to 3, characterised in that the alkylating agent in reaction step b) is methyl iodide.

5. Process according to Claims 1 to 4, characterised in that the chlorinating agent in reaction step f) is chlorine or sulphuryl chloride.

**Revendications**

1. Procédé de production d'acides 7-amino-1-déthia-1-oxa-3-hydroxyméthyl-céphem-4-carboxyliques et de leurs dérivés de formule générale (I)

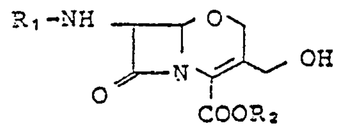

(I)

et de leurs lactones de formule générale (II)

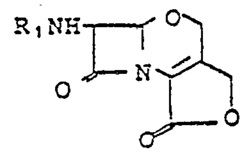

(II)

et de leurs isomères de position de la double liaison de formule générale (III)

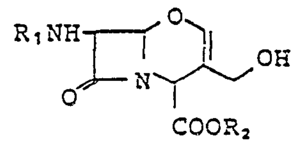

(III)

formules dans chacune desquelles

$R_1$ est l'hydrogène ou un reste acyle d'un acide carboxylique organique classique dans des céphalosporines ou le groupe benzyloxycarbonyle éventuellement substitué et

$R_2$ est un groupe protégeant la fonction carboxyle ou un reste ester pouvant être utilisé du point de vue pharmaceutique, caractérisé en ce que

a) on fait réagir un composé de formule générale (IV)

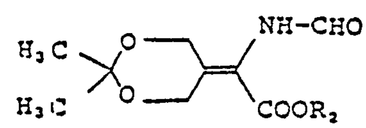

(IV)

dans laquelle

$R_2$ a la définition indiquée ci-dessus, dans un solvant avec un agent de sulfuration pour former des composés de formule générale (V)

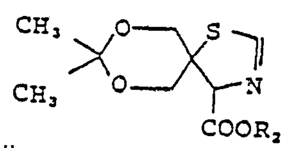

(V)

dans laquelle

$R_2$ a la définition indiquée ci-dessus,

b) on transforme ensuite ces composés avec une base forte dans un solvant à basses températures, puis avec un agent d'alkylation, en composés de formule générale (VI)

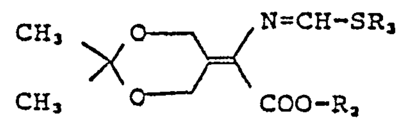

(VI)

ou en leurs isomères de position de la double liaison de formule (VI')

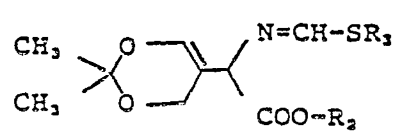

(VI')

dans laquelle

$R_2$ a la définition indiquée ci-dessus et

$R_3$ désigne un groupe alkyle éventuellement substitué, ou bien on transforme des composés de formule générale (IV') ou (IV'')

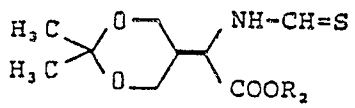

(IV')

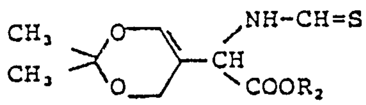

(IV'')

dans lesquels

$R_2$ a la définition indiquée ci-dessus, avec un agent d'alkylation en composés de formule générale (VI) ou (VI'),

c) on transforme ensuite ces composés avec un dérivé activé d'un acide de formule générale (VII)

$$X-CH_2-COOH \qquad (VII)$$

dans laquelle

X est un groupe azido ou phthalimido, en présence d'une base dans un solvant en composés de formule générale (VIII)

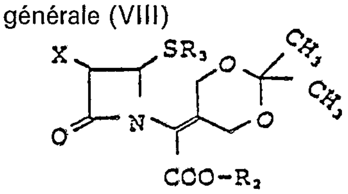

(VIII)

ou en leurs isomères de position de la double liaison, de formule (VIII')

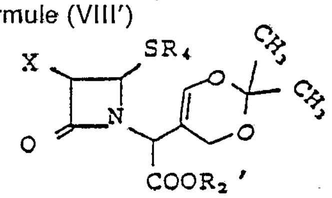

(VIII')

$R_2$, $R_3$ et X ayant les définitions indiquées ci-dessus,

d) on transforme ensuite ces composés, au cas où X est un groupe azide, avec un agent réducteur en présence d'une amine dans un solvant, ou au cas où X est un groupe phtalimido, avec des réactifs cédant du phtalimide, en composés de formule générale (IX)

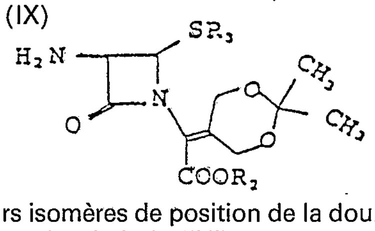

(IX)

ou en leurs isomères de position de la double liaison de formule générale (IX')

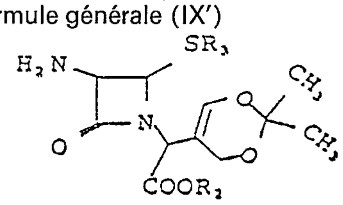

(IX')

$R_2$ et $R_3$ ayant les définitions indiquées ci-dessus,

e) on fait ensuite réagir ces composés avec un acide carboxylique activé sur le groupe carboxyle, de formule $R_1'-COOH$, dans laquelle $R_1'$, désigne un reste organique ou on les transforme ensuite par réaction avec des réactifs qui permettent d'introduire le groupe benzyloxycarbonyle éventuellement substitué, en composés de formule générale (X)

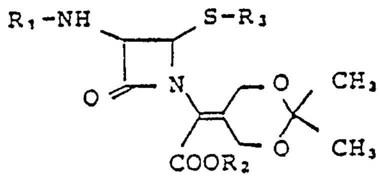

(X)

ou en leurs isomères de position de la double liaison de formule générale (X')

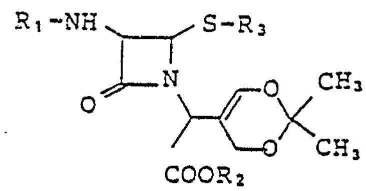

(X')

dans lesquels

$R_1$, $R_2$ et $R_3$ ont les définitions indiquées ci-dessus et

f) on transforme ensuite les composés de formule générale (X) avec un agent de chloration, dans un solvant inerte vis-à-vis de la chloration, puis avec un acide protonique ou avec un acide de Lewis dans le même solvant, en composés de formule générale (I) ou (II) et on transforme les composés de formule générale (X') avec un agent de chloration dans un solvant inerte vis-à-vis de la chloration puis avec un acide protonique ou avec un acide de Lewis en composés de formule générale (III).

2. Procédé suivant la revendication 1, caractérisé en ce que l'agent de sulfuration utilisé dans la transformation du composé (IV) en composé (V) est le réactif de Lawesson.

3. Procédé suivant l'une des revendications 1 ou 2, caractérisé en ce que la base forte dans l'étape réactionnelle b) est le butyllithium.

4. Procédé suivant les revendications 1 à 3, caractérisé en ce que l'agent d'alkylation dans l'étape réactionnelle b) est l'iodure de méthyle.

5. Procédé suivant les revendications 1 à 4, caractérisé en ce que l'agent de chloration dans l'étape réactionnelle f) est le chlore ou le chlorure de sulfuryle.